(19) Europäisches Patentamt
European Patent Office
Office européen des brevets

(11) Veröffentlichungsnummer: **0 204 111**
**A2**

(12) **EUROPÄISCHE PATENTANMELDUNG**

(21) Anmeldenummer: **86105100.1**

(22) Anmeldetag: **14.04.86**

(51) Int. Cl.⁴: **C 07 C 79/35**
C 07 D 307/12, C 07 C 76/02

(30) Priorität: **04.06.85 DE 3519982**

(43) Veröffentlichungstag der Anmeldung:
**10.12.86 Patentblatt 86/50**

(84) Benannte Vertragsstaaten:
**BE CH DE FR GB LI**

(71) Anmelder: **CASSELLA Aktiengesellschaft**
**Hanauer Landstrasse 526**
**D-6000 Frankfurt am Main 61(DE)**

(72) Erfinder: **Schophoff, Friedrich, Dr.**
**Konstanzer Strasse 64**
**D-6000 Frankfurt am Main 61(DE)**

(74) Vertreter: **Urbach, Hans-Georg, Dr. et al.**
**Hanauer Landstrasse 526**
**D-6000 Frankfurt am Main 61(DE)**

(54) **Verfahren zur Herstellung von 2,4-Dinitrophenylethern.**

(57) 2,4-Dinitrophenylether der Formel I

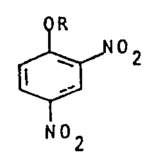

(I)

wobei R z.B. $(C_1-C_4)$Alkoxy-$(C_2-C_4)$alkyl, bedeutet, werden durch Umsetzung eines 2,4-Dinitrochlor- oder -brombenzols mit einem Alkohol ROH in Gegenwart eines Alkalihydroxids hergestellt, wobei die umsetzung bei einem Molverhältnis 2,4-Dinitrochlor- oder -brombenzol zu Alkohol ROH von 1 : (7 bis 25) und bei einer Reaktionstemperatur von −25°C bis +50°C durchgeführt wird.

EP 0 204 111 A2

# Verfahren zur Herstellung von 2,4-Dinitrophenylethern

Die Erfindung betrifft ein Verfahren zur Herstellung von 2,4-Dinitrophenylethern der allgemeinen Formel I

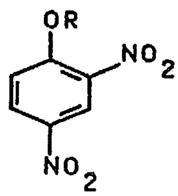

(I)

wobei

R = $(C_1-C_4)$Alkoxy-$(C_2-C_4)$alkyl, Phenoxy-$(C_2-C_4)$alkyl, $(C_1-C_4)$Alkoxy-$(C_2-C_4)$alkoxy-$(C_2-C_4)$alkyl oder Tetrahydrofurfuryl

bedeutet.

Die Verbindungen der Formel I sind wichtige Vorprodukte, z.B. zur Herstellung von Dispersionsfarbstoffen, wie sie z.B. in der belgischen Patentschrift 634 032 beschrieben sind.

Für die Herstellung der Ether der Formel I bieten sich als Ausgangsprodukte z.B. 2,4-Dinitrochlorbenzol und die entsprechenden Alkohole an. Zur Herstellung von 1-(2-Methoxyethoxy)-2,4-dinitrobenzol ist es aus Journal Organic Chemistry USSR 18, (1982), Seite 1087, bekannt, 2,4-Dinitrochlorbenzol mit Natrium-2-methoxy-ethanolat in 2-Methoxyethanol 4 h bei 90°C umzusetzen. Als erhaltene Ausbeute werden dabei jedoch lediglich 62 % angegeben.

Zur Herstellung von 2,4-Dinitro-anisol ist es aus Beispiel 23 der DE-OS 26 34 419 bekannt, 2,4-Dinitrochlorbenzol und Methanol in Gegenwart einer wäßrigen Lösung von Natriumhydroxid und einer wäßrigen Lösung von Benzyldimethyl-lauryl-ammoniumchlorid als Phasentransferkatalysator umzusetzen. Die Umsetzung von 2,4-Dinitrochlorbenzol mit Methanol in Gegenwart von Natriumhydroxid

kann bei Rückflußtemperatur nach der Stufe 1 des Beispiels 1 der EP-PS 0011048 auch ohne Anwesenheit eines Phasentransferkatalysators durchgeführt werden. Bei der Übertragung dieses Verfahrens auf Alkohole der Formel ROH, wobei R die eingangs genannte Bedeutung besitzt, werden jedoch die gewünschten Ether zum Teil nur in mäßigen Ausbeuten erhalten, vor allem treten dabei aber unerwünschte Nebenprodukte, insbesondere 2,4-Dinitrophenol in Mengen von 7 bis 20% auf.

Hierdurch wird eine aufwendige Abtrennung dieses unerwünschten und störenden Nebenproduktes zwingend erforderlich, weil in Gegenwart von 2,4-Dinitrophenol bei der weiteren Umsetzung (z.B. Reduktion) der gewünschten Phenolether technisch nicht beherrschbare Probleme der Produktqualität und der Reaktionsführung auftreten, so daß ein angestrebtes Eintopfverfahren auf dieser Basis nicht realisierbar ist. Darüberhinaus gestaltet sich die Abtrennung und Beseitigung des 2,4-Dinitrophenols aufgrund seiner hohen Toxizität ökologisch wie ökonomisch aufwendig.

Es wurde nun überrascherweise gefunden, daß sich bei der Herstellung von 2,4-Dinitrophenylethern der allgemeinen Formel I durch Umsetzung eines 2,4-Dinitrohalogenbenzols der allgemeinen Formel II

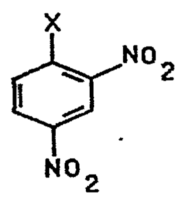

(II)

wobei X = -Cl oder -Br bedeutet, mit einem Alkohol der allgemeinen Formel III

ROH (III)

wobei R die bereits genannte Bedeutung besitzt, in Gegenwart eines Alkalihydroxids die Bildung unerwünschter Nebenprodukte, insbesondere von 2,4-Dinitrophenol erheblich verringern und die Ausbeute an 2,4-Dinitrophenylether steigern läßt, wenn bei der Umsetzung ein Molverhältnis 2,4-Dinitrohalogenbenzol der Formel II zu Alkohol ROH der Formel III von 1 : (7 bis 25) und eine Reaktionstemperatur von -25°C bis +50°C eingehalten wird.

Das Molverhältnis zwischen dem 2,4-Dinitrohalogenbenzol der Formel II und dem Alkohol ROH der Formel III beträgt vorzugsweise 1 : (10 bis 25). Höhere molare Überschüsse an Alkohol ROH als 1 : 25 einzusetzen, ist zwar möglich, bringt aber keine weiteren Vorteile.

Das erfindungsgemäße Verfahren wird vorzugsweise bei Temperaturen von -5 bis +25°C, und ganz besonders bevorzugt von 0 bis +7°C, durchgeführt.

Als Alkalimetallhydroxid kann z.B. Natrium-, Kalium- oder Rubidiumhydroxid verwendet werden. Üblicherweise wird Natriumhydroxid oder Kaliumhydroxid eingesetzt, von denen normalerweise Natriumhydroxid aus Kostengründen bevorzugt wird. Auch ein Gemisch verschiedener Alkalimetallhydroxide kann verwendet werden. Das Molverhältnis 2,4-Dinitrohalogenbenzol : Alkalimetallhydroxid beträgt normalerweise 1 : (1 bis 1,2), vorzugsweise 1 : (1 bis 1,1). Das Alkalimetallhydroxid wird bei dem erfindungsgemäßen Verfahren in der Regel in wasserfreier Form eingesetzt.

Die Alkoxy- und Alkyl-gruppen in den für R stehenden Resten können geradkettig oder verzweigt sein. Als Alkohole der Formel III können z.B. eingesetzt werden: Methylglykol (= Ethylenglykolmonomethylether = $CH_3OCH_2CH_2OH$, die folgenden Bezeichnungen sind analog gebildet), Ethylglykol, Propylglykol, Isopropylglykol,

Butylglykol, Phenylglykol; Methyldiglykol (= Diethylenglykolmonomethylether = $CH_3OCH_2CH_2OCH_2CH_2OH$, die folgenden Bezeichnungen sind analog gebildet), Ethyldiglykol, Isopropyldiglykol, Butyldiglykol, Isobutyldiglykol; 2- oder 3-Methoxy-propanol, 2- oder 3-Propoxy-propanol, 2- oder 3-Isopropoxy-propanol, 2- oder 3-Isobutoxy-propanol, 2-, 3- oder 4-Methoxy-butanol, 2-, 3- oder 4-Isopropoxy-butanol, 2-, 3-oder 4-Butoxy-butanol, 2-, 3- oder 4-Isobutoxy-butanol; 4,8-Dioxanonan-1-ol, 4,8-Dioxadecan-1-ol, 4,8-Dioxaundecan-1-ol, 5,10-Dioxaundecan-1-ol, 5,10-Dioxadodecan-1-ol, 3,6-Dioxa-2,5-dimethyl-heptan-1-ol, 3,6-Dioxa-2,5-dimethyl-nonan-1-ol, 3,6-Dioxa-2,5-diethyl-octan-1-ol, Tetrahydrofurfurylalkohol.

Als Alkohol der Formel III wird vorzugsweise Methylglykol (= Ethylenglykolmonomethylether) verwendet.

Wenn als Alkohol der Formel III ein Gemisch von zwei oder mehr Alkoholen der Formel ROH eingesetzt wird, wobei R die eingangs genannte Bedeutung besitzt, dann entstehen Gemische aus zwei oder mehr Verbindungen der Formel I.

Bei der Durchführung des erfindungsgemäßen Verfahrens kann das Alkalimetallhydroxid zu einer Lösung des 2,4-Dinitrohalogenbenzols der Formel II in dem Alkohol ROH der Formel III zudosiert werden. Es ist auch möglich, das 2,4-Dinitrohalogenbenzol der Formel II und das benutzte Alkalimetallhydroxid getrennt in dem Alkohol ROH der Formel III zu lösen und die so getrennt hergestellten Lösungen anschließend bei den bereits genannten Temperaturen langsam zu vereinigen. Dabei legt man vorzugsweise die Lösung des 2,4-Dinitrohalogenbenzols vor und dosiert die Lösung des Alkalimetallhydroxids in dem Alkohol ROH der Formel III langsam unter Rühren zu. (Bei der Auflösung des Alkalimetallhydroxids in dem Alkohol ROH bildet sich in einer Gleichgewichtsreaktion das entsprechende Alkalimetallalkoholat und Wasser.) Nach der Beendigung

der Zudosierung wird normalerweise bis zur Beendigung der Umsetzung noch ca. 10 bis 50 min nachgerührt. Die Gesamtreaktionszeiten, vom Beginn der Zudosierung an gerechnet, bis zur Beendigung der Reaktion, betragen normalerweise 1 bis 8 Stunden, vorzugsweise 2 bis 5 Stunden.

Das nach Beendigung der Umsetzung anfallende Reaktionsgemisch kann nach Entfernen des ausgefallenen Alkalimetallhalogenids entweder ohne Zwischenisolierung für nachfolgende Reaktionen eingesetzt werden oder nach an sich bekannten Verfahren aufgearbeitet werden.

Beispielsweise kann zur Isolierung des Ethers der Formel I der überschüssige Alkohol der Formel III, nach der Beseitigung eines eventuell vorhandenen Basenüberschusses durch Zugabe einer Säure, unter vermindertem Druck abdestilliert werden.

Bei dem erfindungsgemäßen Verfahren werden hohe Ausbeuten an Ethern der Formel I erhalten, die durchwegs bei 97 % oder darüber liegen. Die Gehalte an Nebenprodukten, insbesondere an 2,4-Dinitrophenol, liegen unter 1,8 % und sinken auf vernachlässigbare Werte unter 0,1 %, wenn die Reaktion bei Temperaturen unter +25°C durchgeführt wird. Die Ausbeutesteigerung und drastische Reduzierung der Nebenproduktbildung waren, insbesondere in ihren Ausmaßen, nicht vorhersehbar.

In den nachfolgenden Beispielen sind Temperaturen in Celsiusgraden angegeben. Sofern nichts anderes angegeben ist, bedeuten Teile Gewichtsteile und Prozente Gewichtsprozente.

<u>Beispiel 1</u>

Eine Lösung von 40 g Ätznatron (NaOH) in 1141 g Methylglykol wird bei 0 bis 5°C innerhalb von 2,5 h zu einer gerührten Lösung von 202,6 g 2,4-Dinitrochlorbenzol in 380 g Methylglykol getropft. Man rührt noch 30 min bei 0

bis 5°C und filtriert dann vom ausgefallenen NaCl ab.

Die Lösung kann in dieser Form zu weiteren Reaktionen eingesetzt werden.

Zur Isolierung des Produkts wird das überschüssige Methylglykol im Wasserstrahlvakuum abdestilliert. Man erhält 238.6 g (98,6 % der Theorie) β-Methoxyethoxy-2,4-dinitrobenzol als hellgelbe Kristallmasse mit einem Fp von 38°C. Die gaschromatographisch ermittelte Reinheit des Produkts beträgt mindestens 99,5 %. Der Gehalt an 2,4-Dinitrophenol liegt unter 0,1 %.

<u>Beispiel 2</u>
Bei einer Wiederholung des Beispiels 1 werden anstelle des Ätznatrons 56,1g KOH eingesetzt.

Nach der Isolierung erhält man 238,1 g (98,3 % der Theorie) β-Methoxyethoxy-2,4-dinitrobenzol, das im Schmelzpunkt und Reinheitsgrad mit dem nach Beispiel 1 erhaltenen Material übereinstimmt.

<u>Beispiel 3</u>
Arbeitet man, wie in Beispiel 1 angegeben, bei einer Reaktionstemperatur von -20°C, erhält man nach einer Reaktionszeit von insgesamt 5 h 239,0 g (98,8 % der Theorie) β-Methoxyethoxy-2,4-dinitrobenzol, das im Schmelzpunkt und Reinheitsgrad mit dem nach Beispiel 1 erhaltenen Material übereinstimmt.

<u>Beispiel 4</u>
Bei einer Wiederholung des Beispiels 1 wird das Ätznatron in 342 g Methylglykol und das 2,4-Dinitrochlorbenzol in 228g Methylglykol gelöst. Nach einer Reaktionszeit von 3,5 h bei einer Temperatur von 10°C werden 238,3 g (98,5 % der Theorie) β-Methoxyethoxy-2,4-dinitrobenzol mit einem Fp von 37 bis 38°C erhalten. Der gaschromatographisch ermittelte 2,4-Dinitrophenolgehalt des Produktes liegt unter 0,1 %.

Beispiel 5

Man versetzt eine Lösung von 202,6 g 2,4-Dinitrochlorbenzol in 380 g Methylglykol innerhalb von 2 h bei 40°C mit einer Lösung von 42 g Ätznatron (NaOH) in 760 g Methylglykol. Man rührt weitere 30 min bei dieser Temperatur, gibt 2,2 g Oxalsäure zu und entfernt überschüssiges Methylglykol durch Abdestillieren im Wasserstrahlvakuum. Der Rückstand wird mit 600 ml warmem Wasser (40°C) versetzt und unter Rühren auf 20°C abgekühlt. Man erhält nach Absaugen und Trocknen 234,7 g (97 % der Theorie) β-Methoxyethoxy-2,4-dinitrobenzol als hellgelbes Granulat vom Fp 37 bis 38°C. Die gaschromatographisch ermittelte Reinheit beträgt mindestens 99,3 %, der 2,4-Dinitrophenolgehalt liegt bei 0,09 %. In der wäßrigen Phase lassen sich durch HPLC-Analyse 0,42 % 2,4-Dinitrophenol (= insgesamt 2,94 g pro Ansatz, 1,6 % der Theorie) nachweisen.

Beispiel 6

Man arbeitet, wie in Beispiel 5 angegeben, setzt jedoch 48 g NaOH und 9 g Oxalsäure ein und führt die Umsetzung bei einer Temperatur von -5°C durch. Man erhält nach einer Reaktionszeit von insgesamt 4 h 237,4 g (98,1 % der Theorie) β-Methoxyethoxy-2,4-dinitrobenzol, das in Schmelzpunkt und Reinheitsgrad mit dem nach Beispiel 1 erhaltenen Material übereinstimmt. In der wäßrigen Phase lassen sich durch HPLC-Analyse 0,11 % Dinitrophenol nachweisen, was einem 2,4-Dinitrophenolanfall pro Ansatz von 0,74 g (0,4 % der Theorie) entspricht.

Beispiel 7 (Vergleichsbeispiel)

Analog der Reaktionsstufe 1 der EP-PS 0011048 werden 213 g geschmolzenes 2,4-Dinitrobenzol in 632 g Methylglykol eingebracht und unter Rühren gelöst. Zu der gerührten

Lösung werden 86 g 50%ige wäßrige NaOH-Lösung bei 65°C zugetropft. Anschließend wird eine Stunde bei 65°C nachgerührt.

Das erhaltene Reaktionsgemisch enthält nach der HPLC-Analyse β-Methoxyethoxy-2,4-dinitrobenzol und 2,4-Dinitrophenol im Gewichtsverhältnis 73 : 21 sowie 6 Teile nicht näher identifizierte Nebenprodukte. Der Ansatz wird mit 800 g Eis versetzt und das ausgefallene Produkt abgesaugt und mit 2 l kaltem Wasser gewaschen. Man erhält 193 g (75 % der Theorie) eines dunkelbraunen, Nebenprodukte enthaltenden Öls (gaschromatographisch bestimmter Reingehalt 94 %). Aus der wäßrigen Phase werden durch Ansäuern auf pH 1 36,7 g (19 %) 2,4-Dinitrophenol erhalten.

<u>Beispiel 8</u> (Vergleichsbeispiel):
Bei einer Wiederholung des Beispiels 7 werden anstelle wäßriger NaOH-Lösung 42 g festes NaOH eingesetzt.

Es werden 229,2 g (= 90,2 % der Theorie) β-Methoxyethoxy-2,4-dinitrobenzol als dunkelbrauner Feststoff vom Fp: 31 bis 33°C erhalten, der einen gaschromatographisch bestimmten Reingehalt von 97 % besitzt. Aus der wäßrigen Phase werden durch Ansäuern auf pH 1 13,5 g (7 %) 2,4-Dinitrophenol isoliert.

<u>Beispiel 9</u> (Vergleichsbeispiel)
Analog der Reaktionsstufe 1 des Beispiels 9 der EP-PS 0011048 werden zu 632 g Methylglykol bei Temperaturen unter 50°C innerhalb von 15 bis 20 Minuten 42 g festes Ätznatron zugegeben. Hierzu werden in 5 bis 20 Minuten 213 g 2,4-Dinitrochlorbenzol bei Temperaturen unter 50°C gegeben. Das Reaktionsgemisch wird zum Rückfluß erhitzt und 3 h unter Rückfluß gehalten. Die erhaltene Lösung enthält nach HPLC-Analyse β-Methoxyethoxy-2,4-dinitrobenzol, 2,4-Dinitrophenol, 2,4-Dinitrochlorbenzol und

nicht näher identifizierte Nebenprodukte im Gewichtsverhältnis 81 : 11 : 5 : 3.

Beispiel 10

Bei einer Wiederholung des Beispiels 1 werden statt des Methylglykols ingesamt 2764 g Phenylglykol eingesetzt.

Es werden 298,1 g (= 98 % der Theorie) β-Phenoxyethoxy-2,4-dinitrobenzol vom Fp: 61 bis 62°C mit einem gaschromatographisch ermittelten Reingehalt von 99,3 % erhalten. Der Gehalt an 2,4-Dinitrophenol liegt unter 0,1 %.

Beispiel 11

Bei einer Wiederholung des Beispiels 1 werden statt des Methylglykols ingesamt 2404 g Methyldiglykol (= Diethylenglykolmonomethylether) eingesetzt.

Es werden 277,9 g (= 97,1 % der Theorie) 2-(2-Methoxyethoxy)-ethoxy-2,4-dinitrobenzol vom Fp: 44 bis 45°C und einem gaschromatographisch ermittelten Reingehalt von 98,7 % erhalten. Der Gehalt an 2,4-Dinitrophenol liegt unter 0,1 %.

Beispiel 12

Bei einer Wiederholung des Beispiels 1 werden statt des Methylglykols ingesamt 2081 g 3-Methoxy-butanol eingesetzt.

Es werden 262 g (= 97 % der Theorie) (3-Methoxy-butoxy)-2,4-dinitrobenzol vom Fp: 42 bis 43°C und einem gaschromatographisch ermittelten Reingehalt von 99,0 % erhalten. Der Gehalt an 2,4-Dinitrophenol liegt unter 0,1 %.

Beispiel 13

Bei einer Wiederholung des Beispiels 1 werden statt des Methylglykols ingesamt 2042 g Tetrahydrofurfurylalkohol

eingesetzt.

Es werden 260,6 g (= 97,2 % der Theorie) (Tetrahydro-furfuryloxy)-2,4-dinitrobenzol vom Fp:53 bis 54°C und einer gaschromatographisch ermittelten Reinheit von 98,2 % erhalten. Der Gehalt an 2,4-Dinitrophenol liegt unter 0,1 %.

PATENTANSPRÜCHE

1. Verfahren zur Herstellung von 2,4-Dinitrophenylethern der allgemeinen Formel I

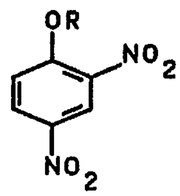
(I)

wobei R = $(C_1-C_4)$Alkoxy-$(C_2-C_4)$alkyl, Phenoxy-$(C_2-C_4)$-alkyl, $(C_1-C_4)$Alkoxy-$(C_2-C_4)$alkoxy-$(C_2-C_4)$alkyl oder Tetrahydrofurfuryl

bedeutet, durch Umsetzung eines 2,4-Dinitrohalogenbenzols der allgemeinen Formel II

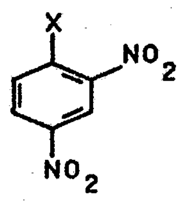
(II)

wobei X = -Cl oder -Br bedeutet, mit einem Alkohol der allgemeinen Formel III

ROH (III)

wobei R die bereits genannte Bedeutung besitzt, in Gegenwart eines Alkalihydroxids, dadurch gekennzeichnet, daß die Umsetzung bei einem Molverhältnis 2,4-Dinitrohalogenbenzol der Formel II zu Alkohol ROH der Formel III von 1 : (7 bis 25) und bei einer Reaktionstemperatur von -25°C bis +50°C durchgeführt wird.

2. Verfahren nach Anspruch 1, dadurch gekennzeichnet, daß das Molverhältnis von 2,4-Dinitrohalogenbenzol der Formel

II und dem Alkohol ROH der Formel III 1 : (10 bis 20) beträgt.

3. Verfahren nach Anspruch 1 und/oder 2, dadurch gekennzeichnet, daß es bei einer Reaktionstemperatur von -5 bis +25°C durchgeführt wird.

4. Verfahren nach einem oder mehreren der Ansprüche 1 bis 3, dadurch gekennzeichnet, daß es bei einer Reaktionstemperatur von 0 bis +7°C durchgeführt wird.

5. Verfahren nach einem oder mehreren der Ansprüche 1 bis 4, dadurch gekennzeichnet, daß das 2,4-Dinitrohalogenbenzol der Formel II und das Alkalimetallhydroxid im Molverhältnis 1 : (1 bis 1,2), vorzugsweise 1 : (1 bis 1,1), eingesetzt werden.

6. Verfahren nach einem oder mehreren der Ansprüche 1 bis 5, dadurch gekennzeichnet, daß als Alkalimetallhydroxid Kaliumhydroxid oder vorzugsweise Natriumhydroxid verwendet wird.

7. Verfahren nach einem oder mehreren der Ansprüche 1 bis 6, dadurch gekennzeichnet, daß als 2,4-Dinitrohalogenbenzol der Formel II 2,4-Dinitrochlorbenzol verwendet wird.

8. Verfahren nach einem oder mehreren der Ansprüche 1 bis 7, dadurch gekennzeichnet, daß als Alkohol der Formel III Methylglykol (=Ethylenglykolmonomethylether) verwendet wird.